Europäisches Patentamt

European Patent Office

Office européen des brevets

(11) Publication number: **0 337 969**
**A1**

## (12) EUROPEAN PATENT APPLICATION

(21) Application number: **89850105.1**

(22) Date of filing: **04.04.89**

(51) Int. Cl.⁴: **A 41 B 13/02**

(30) Priority: **11.04.88 SE 8801323**

(43) Date of publication of application:
**18.10.89 Bulletin 89/42**

(84) Designated Contracting States: **GR**

(71) Applicant: **Mölnlycke AB
S-405 03 Göteborg (SE)**

(72) Inventor: **Kling, Robert
Vävaregatan 13
S-511 63 Skene (SE)**

(74) Representative: **Hammar, Ernst et al
H. ALBIHNS PATENTBYRA AB P.O. Box 3137
S-103 62 Stockholm (SE)**

(54) **A disposable diaper.**

(57) The present invention relates to a disposable diaper which has transversally extending and pretensioned elastication (10) provided along at least one end part of the diaper.

According to the invention, the elastication (10) is positioned on the side of the diaper which faces the wearer is use and is joined to a transverse flap (11) which can be swung along at least a part of its length about an attachment line (13) which extends parallel with the elastication (10) and which is located inwardly of the one edge (15) of the absorption pad, on the side facing the wearer when the diaper is worn.

FIG. 3

EP 0 337 969 A1

## Description

## A disposable diaper.

The present invention relates to disposable diaper of the kind which comprises an absorption body, or pad, and a casing which envelops the absorption pad and which comprises a liquid permeable layer, which is intended to face the wearer, and a liquid impermeable backing layer, which is located on the opposite side of said pad, and which further comprises pretensioned elastication which extends transversally to the longitudinal extension of the diaper and which is disposed along at least one end part of the diaper.

Diapers of the kind in which an absorption and a liqid impervious plastic envelope or casing are combined to form a unit, a so-called all-in-one-diaper, are normally provided with elastication at those regions of the diaper where leakage or seepage is most likely to occur, i.e. those parts of the diaper which are intended to embrace the thighs or waist of the wearer. Such diapers are also provided with fastener means, so called adhesive tabs, thereby obviating the need for tie pants or diaper panties in securing the diaper in position on the wearer. Thus, all-in-one-diapers are a combination of diaper and diaper pants. However, despite the improvement afforded by the combination over early known diapers, the all-in-one-diaper nevertheless lacks the flexibility, suppleness, elasticity and mouldability of conventional underpants, panties or corresponding garment. This deficiency is due to the fact that the absorption body or pad, which is normally comprised mostly of fluff pulp, is relatively thick and rigid and consequently tends to wrinkle and form folds, instead of conforming smoothly to the contours of the wearer's body.

It is known to improve the mouldability, i.e. the ability of the diaper to mould to the contours of the wearer's body, while at the same time preventing the leakage of urine, by applying some form of elastication along the marginal surfaces adjacent the crotch area of the diaper. Such provision results in an elastic seal around the thighs of the wearer. A diaper of this kind is described in SE 7905765-9. However, the provision of an elastic seal around the thighs of the wearer is not sufficient to achieve satisfactory protection against the leakage of urine, particularly when the wearer is an infant and is therefore often in a recumbent position or creeping on hands and knees. In cases such as these, urine is able to run readily along the infant's body, up to his/her waist, without being absorbed by the diaper, since the diaper will not always be in close, intimate contact with the infant's stomach or back, due to the relative inflexibility of the absorption pad. Thus, because of the stiffness of the absorption pad, gaps through which urine can escape will often form at the waist edge margins of the diaper.

It is known to provide a diaper with an elastic band, elastic threads or like elastication in the waist and back regions of the diaper, between the backing layer or sheet of said diaper at a location beyond the ends of the absorption pad, in order to solve the problem of urine leakage at the waist region of the diaper. Examples of this solution are to be found in US Patent Specification No. 4 515 595 and in European Patent Application No. 155 636. US Patent Specification No. 4 685 916 teaches a diaper in which the waist elastic is placed between the outer sheets, such that the position of the elastic corresponds to a narrow elongated indentation in the short end of the absorption pad. GB 2 173 688 A describes a disposable diaper which is provided with a waist leakage protector which comprises an inelastic part positioned between the absorption pad and the liquid permeable sheet, and an elastic part which is placed externally of the waist edge of the absorption pad, at a location between the liquid permeable sheet and the liquid impermeable backing sheet. However, this arrangement will only provide a satisfactory seal when the elastic is so tightly drawn as to risk chafing the skin of the wearer. When the elastic is loosened, to avoid chafing, the seal obtained is inadequate in preventing the leakage of urine.

Furthermore, the provision of elastication externally of the ends of the absorption pad does not prevent the formation of folds or wrinkles and will not improve contact of the absorption pad with the infant's body. Thus, urine is still able to run in the channels formed by the folds and wrinkles, towards the infant's waist and there collect between the end edges of the absorption pad and the elastication located externally thereof. This collection of urine will exaggerate the unpleasantness and irritation caused by the elastication.

Another drawback with the arrangement of elastications between the liquid permeable sheet and the liquid impermeable sheet of the diaper is that the presence of this elastic inhibits the ability of the diaper to breathe, i.e. impairs the ability of the diaper to allow vapour to pass therethrough. Thus, the elastic element in contact with the liquid permeable sheet will prevent the urine absorbed by the diaper from evaporating to the surroundings, resulting in moisture and heat problems within the diaper.

The object of the present invention is to provide in disposable diapers an elastic waist seal which will eliminate the drawbacks of earlier known waist elastics.

A diaper according to the invention is characterised in that the elastication is placed on that side of the diaper which faces the wearer when the diaper is in use and is secured by means of a transverse flap which can be swung at least along part of its length about an attachment line which extends parallel with the elastication and which is located inwardly of the end edges of the absorption pad, on the side which faces the wearer.

By fastening the elastication on the inside of the diaper, inwardly of the end edges of the absorption pad, the important advantage is afforded that both the outer casing of the diaper and the absorption pad will lie firmly against the body of the wearer, thereby avoiding the aforesaid collection of urine in pocket-like folds between the absorption body and the elastication, where absorption of the urine is not possible and which gives rise to irritation of the skin.

Another advantage afforded by the inventive waist elastic is that the elastic is not glued to the liquid impermeable plastic film folded around the edges of the absorption pad. This enables moisture to evaporate and the vapour to depart quite readily, by using a vapour permeable elastic material, thereby further improving the properties of the diaper and further counteracting irritation of the skin.

Still another advantage afforded by the inventive waist elastic is that the elastication comprises a soft and relatively wide band, which ensures that the diaper will lie softly and tightly against the skin of the wearer.

The invention will now be described in more detail with reference to an exemplifying embodiment thereof illustrated in the accompanying drawings.

Figure 1 is a plan view of a diaper according to the invention with the side of the diaper worn next to the skin being turned towards the viewer.

Figure 2 is a sectional view of the diaper taken on the line II-II in Figure 1.

Figure 3 is a sectional view of the diaper of Figure 1, taken on the line III-III in said Figure.

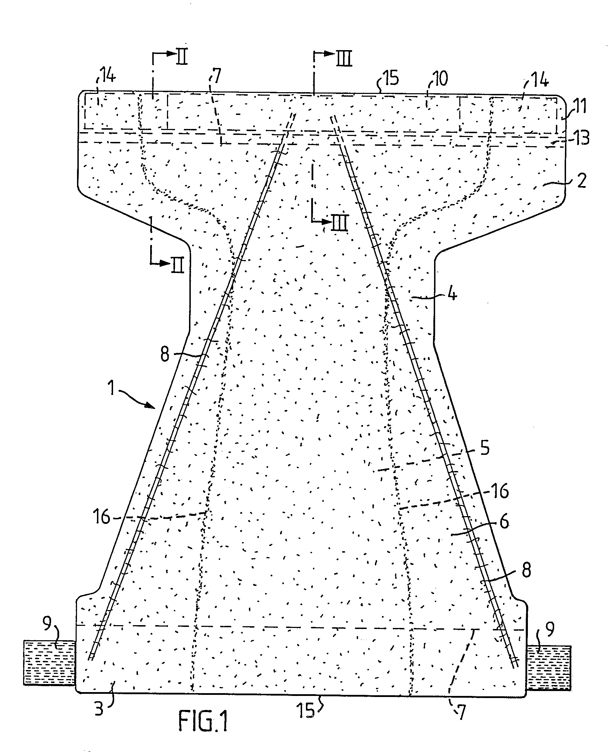

The diaper illustrated in Figure 1 comprises a front part 2, which when the diaper is worn is intended to cover the stomach of the wearer, a back part 3, which is intended to cover the wearer's bottom or buttocks, and a crotch part 4, which is located between said parts 2 and 3. An absorption pad is enveloped in a casing which comprises a liquid permeable sheet 6, which extends laterally beyond the edges 16 of the absorption pad, and a liquid impermeable backing sheet 12 which is located on the opposite side of the of the absorption pad and which also extends laterally beyond the edges of said pad and there joined to the liquid permeable sheet 6. The liquid impermeable backing sheet 12 is folded over the end edges 15 of the diaper and has parts 7 which extend inwards over the end parts of the diaper, on the same side as the permeable sheet 6.

The diaper 1 is also provided with leg elastication 8 comprised of elastic threads which are placed in a V-shaped pattern in the longitudinal direction of the diaper, and also with adhesive tabs 9 for joining the the back part 6 and the front part 2 together when using the diaper. The front diaper part 2 has arranged therein an elastication 10, e.g. a polyurethane foam band or the like, which is inserted beneath a flap 11 on the backing sheet 6 and attached thereto in a pretensioned state. The elastication 10 is embraced by the flap 11 and is secured thereto by means of an adhesive. The two end parts 14 of the flap 11 are secured firmly to the folded-over part 7 of the impermeable backing sheet. Subsequent to be folded around the elastication 10, the flap 11 on the backing sheet 6 is attached to a narrow part 13 of the folded-over part 7, this narrow part extending across the full width of the diaper. The bonds joining the parts 13 and 14 are suitably effected with melt adhesive, which may be applied as a continuous string or intermittently. For example, the flap 11 may be glued to the parts 13,14 along the whole of its length or in some form of pattern, for instance along lines or in punctiform fashion.

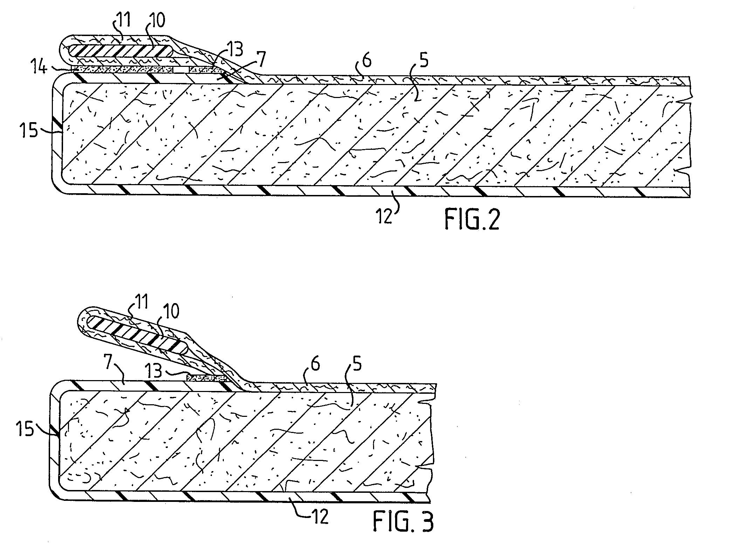

Figure 2 illustrates the flap 11 and its attachment parts 13,14 in section and from one side. The illustrations of Figures 2 and 3 do not show the shapes and sizes of the diaper components in true proportions and the figures shall not therefore be considered as restrictive of the scope of the invention. Figure 3 is a further sectional view of the front part 2 of the diaper, taken on the line III-III in Figure 1. The flap 11 is secured to the liquid impermeable sheet 7, inwardly of the parts 14, solely along the attachment part 13, and consequently the pretensioned elastication 10 is able to move freely in relation to the absorption pad 5 and in relation to the liquid impermeable sheet 7 along the attachment part 13.

It should be mentioned in this connection that it also lies within the purview of the invention to join the flap to the folded-over part 7 solely along the attachment line 13, the attachment at the end parts 14 therewith solely constituting a preferred embodiment.

Two purposes are achieved by allowing the elastic band or strap 10 to move freely about the attachment line 13. Firstly, there is obtained a flexible and pliable seal in the waist region of the wearer, which prevents the leakage of urine and holds the absorption pad in close proximity with skin. Secondly, because the waist elastic is comprised of an air permeable and vapour permeable material, such as polyurethane foam, it will allow moisture to pass therethrough, and because the end parts 14 of said waist elastic are attached in a manner to leave a space between the flap 11 and the folded-over, liquid impermeable part 7, the diaper is enabled to breathe.

By locating the elastic at least partially inwardly of the waist margin of the diaper and the absorption pad, instead of externally of said pad waist margin, less of the wearer's body will be enclosed than when wearing a conventional diaper, since the elastic of the inventive diaper does not reach as far up on the wearer's stomach as the elastic of the conventional diaper. This adds further to the comfort of the inventive diaper.

The elastic band shall have the greatest possible width and will preferably be wider than about 1 cm, so as to provide the largest possible abutment area with the skin and therewith reduce the risk of chafing.

Instead of encapsulating the elastication in the liquid permeable sheet of the diaper, the elastic may be encapsulated in an additional sheet or layer which is subsequently fastened to the liquid permeable sheet.

It will be understood that the described and illustrated embodiments do not restrict the scope of the invention and that modifications can be made within the scope of the following claims.

**Claims**

1. A disposable diaper (1) comprising an absorption body or pad (5) which is enveloped in a casing which includes a liquid permeable sheet (6) which will face the wearer when the diaper is in use, a liquid impermeable backing sheet (12) which is located on the opposite side of the absorption pad (5), and pretensioned elastication (10) which extends transversally to the longitudinal extension of the diaper and which is disposed along at least one end part (2,3) of the diaper; characterised in that the elastication (10) is disposed on the side of the diaper which faces the wearer in use and is joined to a transverse flap (11) which is pivotable along at least a part of its length about an attachment line (13) extending parallel with the elastication (10) and located inwardly of the end edge (15) of the absorption pad on the side which faces the wearer when the diaper is worn.

2. A disposable diaper according to claim 1, characterised in that the distance between the attachment line (13) and the nearest end edge (15) of the absorption pad is greater than about 1 cm.

3. A disposable diaper according to claim 1 or claim 2, characterised in that the elastication (10) is encapsulated in a fold in the liquid permeable sheet (6).

4. A disposable diaper according to claim 1 or claim 2, characterised in the elastication (10) is encapsulated in an additional sheet or layer which lies on the liquid permeable sheet (6).

5. A disposable diaper according to any one of the preceding claims, characterised in that the elastication (10) comprises a soft, vapour permeable material.

6. A disposable diaper according to claim 5, characterised in that the elastication (10) comprises polyurethane foam.

7. A disposable diaper according to claims 1-5, characterised in that the width of the elastication (10) is approximately equal to the distance of the elastication attachment line (13) from the nearest end edge (15) of the absorption pad.

FIG.1

FIG.2

FIG.3

EP 0 337 969 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| A | EP-A1-0 264 238 (THE PROCTER & GAMBLE COMPANY) | 1-7 | A 41 B 13/02 |
| | --- | | |
| A | CH-A5-657 757 (COLGATE-PALMOLIVE COMPANY) | 1-7 | |
| | ----- | | |

TECHNICAL FIELDS SEARCHED (Int. Cl.4)

A 41 B

The present search report has been drawn up for all claims

| Place of search | Date of completion of the search | Examiner |
|---|---|---|
| STOCKHOLM | 16-05-1989 | JÖNSSON H C |